# EUROPEAN PATENT APPLICATION

(11) **EP 4 725 413 A1**
(43) Date of publication of application: **15.04.2026**
(21) Application number: 24911383.8
(22) Date of filing: 26.12.2024
(51) Int. Cl.: A61B 5/145

(54) **SPLIT-TYPE MEDICAL APPARATUS AND PREPARATION METHOD FOR ELECTRONIC ASSEMBLY THEREOF**

(30) Priority: 26.12.2023 CN 202311825600
(71) Applicant: SHENZHEN SISENSING CO., LTD., Shenzhen Guangdong 518110 (CN)
(72) Inventor: LI, Yunfeng, Shenzhen, Guangdong 518110 (CN); WU, Jiang, Shenzhen, Guangdong 518110 (CN)
(74) Representative: Isern Patentes y Marcas S.L.
(86) International application number: PCT/CN2024/142941
(87) International publication number: WO 2025/140462

(57) **Abstract**

The present disclosure relates to a split-type medical apparatus(10) and a method for preparing its electronic assembly(100). The split-type medical apparatus(10) includes a first housing(110), an electronic module(120) and an isolator(130) disposed on the first housing(110), a second housing(200) detachably assembled with the first housing(110), and a sensor(12) disposed on the second housing(200). The second housing(200) includes a mount(210) for the sensor(12) which can be placed subcutaneously; the isolator(130) has a groove-shaped portion(131) matching the mount(210) and a functional surface(132) facing away from the electronic module(120), with a protrusion(133) surrounding the groove-shaped portion(131) provided on the functional surface(132). The electronic module(120) includes a connection area(121) for electrical connection with the sensor(12). The isolator(130) is attached to the first housing(110) and covers the electronic module(120) in such a way that the connection area(121) is exposed in the groove-shaped portion(131); when the first housing(110) is assembled to the second housing(200), the isolator(130) is located between the second housing(200) and the first housing(110), and the protrusion(133) deforms in the direction from the second housing(200) toward the first housing(110). According to the present disclosure, a split-type medical apparatus(10) with improved sealing effect and a method for preparing its electronic assembly(100) can be provided.

## Description

### TECHNICAL FIELD

The present disclosure relates to the field of biomedical engineering industry, and specifically relates to a split-type medical apparatus and a method for preparing its electronic assembly.

### BACKGROUND

Diabetes has currently become one of the major diseases affecting public health. For diabetic patients, real-time monitoring of their own blood glucose changes helps them control the progress of diabetes and supports diabetes treatment. At present, a Continuous Glucose Monitoring (CGM) device is usually used to continuously monitor the blood glucose concentration of the human body. In order to reuse the electronic module of the Continuous Glucose Monitoring device, the existing Continuous Glucose Monitoring device usually adopts a split-type assembly method.

Generally speaking, in order to maintain the normal working performance of the Continuous Glucose Monitoring device, the electronic module needs to be sealed. In the prior art, a split-type assembled Continuous Glucose Monitoring device usually includes an upper housing, a lower housing, and an electronic module disposed between the upper housing and the lower housing, and a sealing ring is usually provided at the junction of the upper housing and the lower housing to seal the electronic module.

However, during the use of the above-mentioned Continuous Glucose Monitoring device in the prior art, the sealing ring may have a risk of loosening, which leads to poor sealing effect on the electronic module, thereby affecting the working performance of the Continuous Glucose Monitoring device.

### SUMMARY OF THE INVENTION

The present disclosure is proposed in view of the above conditions, and its object is to provide a split-type medical apparatus with improved sealing performance and a method for preparing its electronic assembly.

To this end, a first aspect of the present disclosure provides a split-type medical apparatus, which includes a first housing, an electronic module and an isolator disposed on the first housing, a second housing detachably assembled with the first housing, and a sensor disposed on the second housing. The second housing includes a mount for the sensor which can be placed subcutaneously; the isolator has a groove-shaped portion matching the mount and a functional surface facing away from the electronic module, with a protrusion surrounding the groove-shaped portion provided on the functional surface; the electronic module includes a connection area for electrical connection with the sensor, and the isolator is configured to be attached to the first housing and cover the electronic module in such a way that the connection area is exposed from the groove-shaped portion; when the first housing is assembled to the second housing, the isolator is located between the second housing and the first housing, the protrusion deforms in the direction from the second housing toward the first housing, and the mount is located in the groove-shaped portion and the sensor is electrically connected to the electronic module through the connection area.

In the split-type medical apparatus according to the first aspect of the present disclosure, the isolator is attached to the first housing and partially covers the electronic module, which can initially seal the electronic module, and since the isolator has a groove-shaped portion for exposing the connection area and matching the mount for disposing the sensor, when the first housing is assembled to the second housing, the sensor can be electrically connected to the connection area, thereby enabling the split-type medical apparatus to obtain the target physiological information. In addition, since the isolator has a protrusion surrounding the groove-shaped portion, when the first housing is assembled to the second housing, the protrusion surrounding the groove-shaped portion will be squeezed by the second housing and thus deform, so that the isolator can be more closely attached to the second housing when the first housing is assembled to the second housing, and thereby achieving the sealing effect on the groove-shaped portion, that is, sealing the connection area, and further improving the sealing effect on the electronic module.

In addition, in the split-type medical apparatus according to the first aspect of the present disclosure, optionally, the protrusion surrounding the groove-shaped portion is referred to as a first protrusion, the groove-shaped portion has a second protrusion surrounding the connection area, and when the first housing is assembled to the second housing, the second protrusion deforms in the direction from the second housing toward the first housing. In this case, when the first housing is assembled to the second housing, the second protrusion will be squeezed by the mount and thus deform, so that the second protrusion can be more closely attached to the mount, enabling the connection area to be in a sealed space, that is, the interfitting between the second protrusion and the mount can improve the sealing effect on the connection area, thereby improving the sealing effect on the electronic module.

In addition, in the split-type medical apparatus according to the first aspect of the present disclosure, optionally, the split-type medical apparatus includes a power module configured to supply energy to the electronic module; when the groove-shaped portion matching the mount is referred to as a first groove-shaped portion, the isolator has a second groove-shaped portion configured to accommodate the power module, and the protrusion surrounds the first groove-shaped portion and the second groove-shaped portion. In this case, the protrusion surrounds the first groove-shaped portion and the second groove-shaped portion. When the first housing is assembled to the second housing, the protrusion surrounding the first groove-shaped portion and the second groove-shaped portion will be squeezed by the second housing and thus deform, so that the isolator can be more closely attached to the second housing when the first housing is assembled to the second housing, and can seal the connection area located in the first groove-shaped portion and the power module located in the second groove-shaped portion, thereby improving the sealing performance of the split-type medical apparatus.

In addition, in the split-type medical apparatus according to the first aspect of the present disclosure, optionally, the protrusion surrounding the groove-shaped portion is referred to as a first protrusion, a third protrusion connected to the first protrusion and configured to isolate the first groove-shaped portion and the second groove-shaped portion is provided on the functional surface, and in the direction from the first housing toward the second housing, the extension length of the third protrusion is the same as that of the first protrusion. In this case, when the first housing is assembled to the second housing, since the extension length of the first protrusion is the same as that of the third protrusion, the second housing can simultaneously squeeze the first protrusion and the third protrusion, so that both the first protrusion and the third protrusion can be closely attached to the second housing. Through the interfitting of the first protrusion, the third protrusion and the second housing, two independent sealed spaces respectively including the first groove-shaped portion and the second groove-shaped portion can be formed, thereby respectively sealing the connection area and the mount located in the first groove-shaped portion and the power module located in the second groove-shaped portion.

In addition, in the split-type medical apparatus according to the first aspect of the present disclosure, optionally, the mount includes a positioning seat and a base, the positioning seat has a through-hole penetrating the second housing, and the sensor is positioned on the second housing through the through-hole; the base is configured to accommodate a conductive assembly electrically connected to the sensor, the sensor is electrically connected to the connection area through the conductive assembly. When the first housing is assembled to the second housing, the base presses the second protrusion, and the base and the second protrusion cooperate to form a sealed space. In this case, when the first housing is assembled to the second housing, the second protrusion can be squeezed by the base, so that the base can be closely attached to the second protrusion to form a sealed space for accommodating the conductive assembly and the connection area. In addition, the sensor can be disposed on the mount through the through-hole of the positioning seat. When the first housing is assembled to the second housing, the electrical connection between the sensor and the electronic module can be realized through the electrical connection between the conductive assembly and the connection area, and the through-hole penetrates the second housing in the position of the positioning seat, in other words, the through-hole is independent of the sealed space, thereby isolating the undesired substances entering the split-type medical apparatus through the through-hole from the sealed space, and further improving the protection effect on the electrical connection part of the split-type medical apparatus.

In addition, in the split-type medical apparatus according to the first aspect of the present disclosure, optionally, the second housing has at least one hole for positioning the second housing, and when the first housing is assembled to the second housing, the at least one hole is located on the side of the third protrusion away from the second groove-shaped portion. In this case, before the split-type medical apparatus is used, the second housing can be positioned on the component for accommodating the second housing through the hole(s). Since the hole(s) is/are located on the side of the third protrusion away from the second groove-shaped portion, when the first housing is assembled to the second housing, the hole(s) can be independent of the sealed space including the second groove-shaped portion, thereby isolating the undesired substances entering the split-type medical apparatus through the hole(s) from the sealed space including the second groove-shaped portion, and thus improving the sealing effect on the power module.

In addition, in the split-type medical apparatus according to the first aspect of the present disclosure, optionally, at least one fourth protrusion matching the at least one hole is provided on the functional surface, and when the first housing is assembled to the second housing, the at least one fourth protrusion deforms in the direction from the second housing toward the first housing. In this case, the fourth protrusion can block the hole(s), preventing undesired substances from entering the split-type medical apparatus along the hole(s), thereby further improving the sealing performance of the split-type medical apparatus.

In addition, in the split-type medical apparatus according to the first aspect of the present disclosure, optionally, the isolator is elastic and is bonded to the inner contour of the first housing. In this case, after the first housing and the second housing are assembled, since the isolator is elastic, it will rebound after being squeezed, so that the isolator can be more closely attached to the second housing, thereby enhancing the sealing performance of the split-type medical apparatus. Since the isolator is bonded to the first housing, the junction portion between the isolator and the first housing can be closely attached, thereby preventing undesired substances from infiltrating the electronic module through the junction portion.

A second aspect of the present disclosure provides a method for preparing an electronic assembly, wherein the electronic assembly includes the electronic module, the isolator and the first housing according to any one of the first aspect of the present disclosure, and the method for preparing the electronic assembly includes: preparing the first housing provided with the electronic module; coupling the first housing with a mold having a preset shape matching the shape of the isolator; supplying an injection molding material with a preset temperature to the mold; removing the mold after the injection molding material is cooled to obtain the isolator; and obtaining the electronic assembly. In the second aspect of the present disclosure, in the electronic assembly obtained by supplying the injection molding material to the mold, the isolator can be closely bonded to the first housing and seal the electronic module disposed on the first housing. The preset shape of the mold matches the shape of the isolator, so that the injection molding material can form an isolator with a desired structure after cooling, and the structure of the isolator can improve the sealing performance of the electronic assembly after assembly; at the same time, the isolator can also play a role in stabilizing various electronic components of the electronic module.

In addition, in the method for preparing an electronic assembly according to the second aspect of the present disclosure, optionally, the injection molding material includes silicone. In this case, the isolator can have high waterproofness and elasticity, thereby improving the sealing effect on the electronic module. In addition, if the injection molding material includes silicone, the injection molding material can be injected in a low-pressure manner. Since the injection pressure of low-pressure injection is low, the damage to the electronic assembly during the injection molding process can be reduced.

In addition, in the method for preparing an electronic assembly according to the second aspect of the present disclosure, optionally, the preset temperature is 150 degrees Celsius to 200 degrees Celsius. In this case, the injection molding process can be performed with material at a low temperature-thereby further reducing potential damage to the electronic component during the injection molding process.

In addition, in the method for preparing an electronic assembly according to the second aspect of the present disclosure, optionally, the hardness of the injection molding material after curing is 10 Shore A to 60 Shore A. In this case, the isolator can exhibit high elasticity.

According to the present disclosure, a split-type medical apparatus with improved sealing performance and a method for preparing its electronic assembly can be provided.

### BRIEF DESCRIPTION OF THE DRAWINGS

Fig. 1 is a diagram showing an application scenario of a split-type medical apparatus according to an example of the present disclosure.
Fig. 2 is a front view showing the split-type medical apparatus according to an example of the present disclosure.
Fig. 3 is an exploded schematic diagram showing the split-type medical apparatus according to an example of the present disclosure.
Fig. 4A is a structural schematic diagram showing a second housing according to an example of the present disclosure from a first perspective; Fig. 4B is a structural schematic diagram showing the second housing according to an example of the present disclosure from a second perspective.
Fig. 5A is a structural schematic diagram showing an electronic assembly according to an example of the present disclosure from a first perspective; Fig. 5B is a structural schematic diagram showing the electronic assembly according to an example of the present disclosure from a second perspective.
Fig. 6 is a flowchart showing a method for preparing the electronic assembly according to an example of the present disclosure.

### DETAILED DESCRIPTION

Hereinafter, preferred embodiments of the present disclosure will be described in detail with reference to the accompanying drawings. In the following description, the same components are denoted by the same reference numerals, and repeated descriptions will be omitted. In addition, the drawings are only schematic diagrams, and the ratios of dimensions of the components to each other or the shapes of components may be different from actual ones.

The first aspect of the present disclosure relates to a split-type medical apparatus. The split-type medical apparatus according to the present disclosure can be applied to the surface of a user's skin to obtain the user's analyte information, and after processing the analyte information, send the user's analyte information to an intelligent device, thereby enabling monitoring of the user's analyte information. In some examples, the analyte information may also be referred to as the target physiological information. In the split-type medical apparatus according to the present disclosure, the sealing effect on the electronic module can be improved by covering the electronic module with an isolator.

The second aspect of the present disclosure relates to a method for preparing an electronic assembly. The preparation method according to the present disclosure obtains the electronic assembly by supplying an injection molding material to a mold. The isolator in the electronic assembly can be closely bonded to the first housing and seal the electronic module disposed on the first housing, and the structure of the isolator can improve the sealing performance of the electronic assembly after assembly; and at the same time, the isolator can also play a role in stabilizing various electronic components of the electronic module.

The third aspect of the present disclosure relates to a medical instrument suite including the split-type medical apparatus according to the first aspect of the present disclosure.

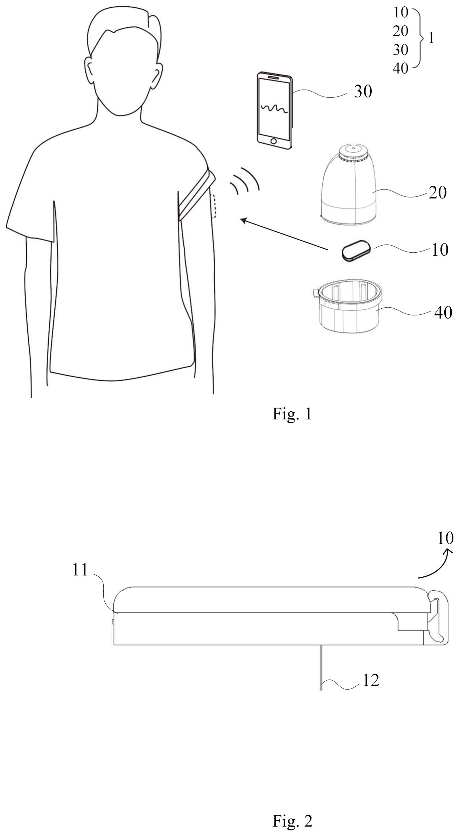

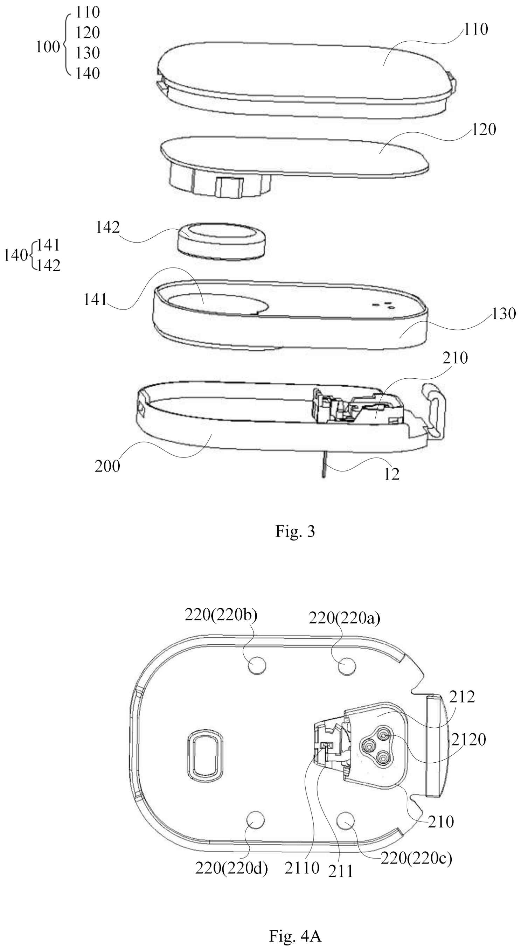

Fig. 1 shows an application scenario diagram of a split-type medical apparatus 10 according to an example of the present disclosure. Fig. 2 shows a front view of the split-type medical apparatus 10 according to the example of the present disclosure. Fig. 3 shows an exploded schematic diagram of the split-type medical apparatus 10 according to the example of the present disclosure.

In some examples, referring to Fig. 1, the medical instrument suite 1 may include the split-type medical apparatus 10 and an application apparatus 20. In some examples, the split-type medical apparatus 10 is a split-type medical apparatus 10 that can be applied to the surface of a target's skin. In some examples, the split-type medical apparatus 10 may also be referred to as a split-type medical instrument or an application assembly. In some examples, the target may be a host with physiological functions.

In some examples, the split-type medical apparatus 10 can be applied to the surface of the host's skin by means of the application apparatus 20. Thereby, the split-type medical apparatus 10 can be applied to a desired position.

In some examples, referring to Fig. 1, the split-type medical apparatus 10 can transmit data information with an intelligent device 30 in a wireless manner. Thereby, the analyte information obtained by the split-type medical apparatus 10 can be read and monitored in real time through the intelligent device 30.

In some examples, the medical instrument suite 1 may include a box apparatus 40 matched with the application apparatus 20. In some examples, the split-type medical apparatus 10 can be assembled by using the application apparatus 20 and the box apparatus 40 matched with the application apparatus 20. Thus, the split-type medical apparatus 10 can be completely assembled into a single unit. In some examples, the medical instrument suite 1 may not include the box apparatus 40, and the split-type medical apparatus 10 may be directly accommodated in the application apparatus 20 in a completely assembled form.

In some examples, referring to Fig. 2, the split-type medical apparatus 10 may include a main body portion 11 and a sensor 12 disposed on the main body portion 11, wherein, the main body portion 11 can be applied to the surface of the skin, and the sensor 12 can be placed subcutaneously. Specifically, the sensor 12 can be inserted subcutaneously into the target. In some examples, the sensor 12 can obtain analyte information, and the main body portion 11 can receive the analyte information and send the analyte information to the intelligent device 30. In this case, real-time monitoring of the analyte information can be realized.

In some examples, referring to Fig. 3, the main body portion 11 may include an electronic assembly 100 and a second housing 200. In some examples, the electronic assembly 100 may include a first housing 110, an electronic module 120 and an isolator 130. In some examples, the electronic module 120 and the isolator 130 may be disposed on the first housing 110. In some examples, the electronic module 120 may be located between the first housing 110 and the isolator 130. Thus, the electronic module 120 can be protected. In some examples, when the electronic assembly 100 is assembled to the second housing 200, the isolator 130 may be located between the second housing 200 and the first housing 110.

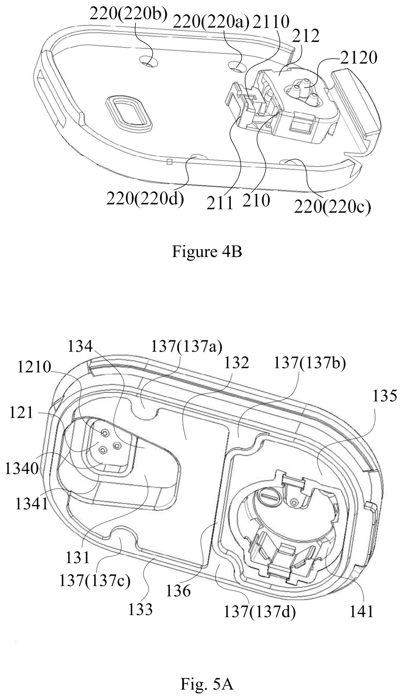

Fig. 4A is a structural schematic diagram showing the second housing 200 according to an example of the present disclosure from a first perspective; Fig. 4B is a structural schematic diagram showing the second housing 200 according to the example of the present disclosure from a second perspective.

In some examples, the second housing 200 can be detachably assembled with the electronic assembly 100. Thus, the flexibility of the split-type medical apparatus 10 can be improved.

In some examples, the second housing 200 can be applied to the surface of the skin. In some examples, the second housing 200 can be detachably assembled with the first housing 110. Thus, it is convenient to install the electronic assembly 100 on the second housing 200 or detach the electronic assembly 100 from the second housing 200.

In some examples, the sensor 12 may be disposed on the second housing 200. Specifically, the second housing 200 may include a mount 210 for disposing the sensor 12 (refer to Fig. 4A or Fig. 4B).

In some examples, referring to Fig. 4A or Fig. 4B, the mount 210 may include a positioning seat 211. In some examples, the positioning seat 211 may be configured to position the sensor 12.

In some examples, the positioning seat 211 may have a through-hole 2110 penetrating the second housing 200. The sensor 12 may be positioned on the second housing 200 through the through-hole 2110. Thus, the sensor 12 can be fixed on the second housing 200.

In some examples, the through-hole 2110 may be used to position the sensor 12, the sensor 12 can be disposed on the mount 210 through the through-hole 2110, and the sensor 12 can pass through the through-hole 2110.

In some examples, the mount 210 may include a base 212. In some examples, the base 212 may be configured to accommodate a conductive assembly 2120 electrically connected to the sensor 12. The sensor 12 may be electrically connected to the electronic module 120 through the conductive assembly 2120. Thus, the electronic module 120 can obtain the analyte information collected by the sensor 12.

In some examples, referring to Fig. 4A or Fig. 4B, the second housing 200 may have at least one hole 220 for positioning the second housing. In some examples, the second housing 200 may be held on the box apparatus 40 through the hole(s) 220. In other words, the second housing 200 may be positioned on the box apparatus 40 through the hole(s) 220. In this case, before the split-type medical apparatus 10 is used, the second housing 200 can be positioned on the component (i.e., the box apparatus 40) for accommodating the second housing 200 through the hole 220.

In some examples, the number of the hole(s) 220 may be one. In some examples, the number of the hole(s) 220 may be multiple. For example, the second housing 200 may have four holes 220 for positioning, which may be hole 220a, hole 220b, hole 220c and hole 220d respectively. Thus, the hole 220a, hole 220b, hole 220c and hole 220d can position the second housing 200 relatively stably. In other words, the hole 220a, hole 220b, hole 220c and hole 220d can position the second housing 200 in the box apparatus 40 relatively stably.

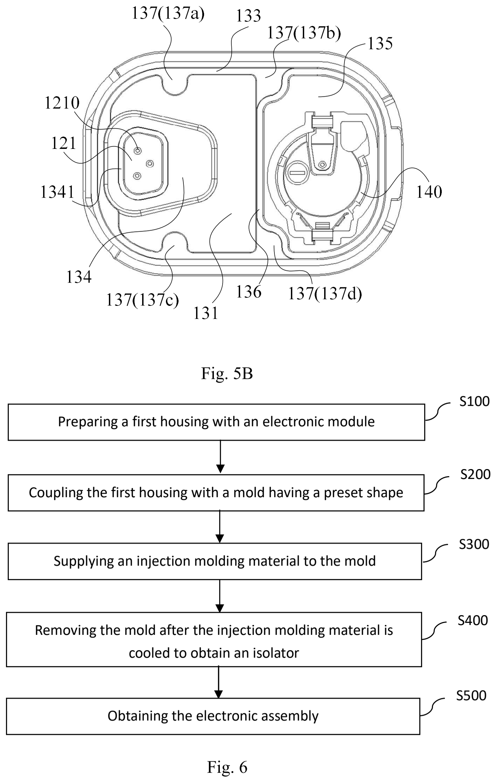

Fig. 5A is a structural schematic diagram showing the electronic assembly 100 according to an example of the present disclosure from a first perspective; Fig. 5B is a structural schematic diagram showing the electronic assembly 100 according to the example of the present disclosure from a second perspective.

In some examples, referring to Fig. 5A or Fig. 5B, the electronic module 120 may be reusable. In other words, the electronic module 120 may be used several times. The electronic module 120 may be disposed on the first housing 110. In this case, the electronic module 120 can be detachably assembled with the second housing 200 along with the first housing 110.

In some examples, the electronic module 120 may be bonded to the first housing 110. Thereby, the stability of the electronic module 120 disposed on the first housing 110 can be improved.

In some examples, the electronic module 120 may receive the analyte information from the sensor 12, process the analyte information, and then send the processed analyte information to the intelligent device 30 in real time.

In some examples, the electronic module 120 may include a connection area 121 electrically connected to the sensor 12 (refer to Fig. 5A or Fig. 5B). In some examples, when the first housing 110 is assembled to the second housing 200, the sensor 12 may be electrically connected to the connection area 121. Thereby, the split-type medical apparatus 10 can obtain the analyte information. In some examples, the sensor 12 may be electrically connected to the connection area 121 through the conductive assembly 2120.

In some examples, the connection area 121 may have electrical contact(s) 1210 that can be electrically connected to the conductive assembly 2120. In this case, when the first housing 110 is assembled to the second housing 200, the electrical connection between the sensor 12 and the electronic module 120 can be realized through the electrical connection between the conductive assembly 2120 and the electrical contact(s) 1210.

As described above, in some examples, the main body portion 11 may further include an isolator 130 (refer to Fig. 3). In some examples, the isolator 130 may be used to seal the electronic module 120. Thus, the electronic module 102 can be protected from undesired substances.

In some examples, the undesired substances may refer to water, sweat, dander, dust, etc.

In some examples, the isolator 130 may be attached to the first housing 110. Thereby, undesired substances can be prevented from entering the position where the electronic module 120 is located from the junction portion of the isolator 130 and the first housing 110. In some examples, the isolator 130 may cover the electronic module 120. Thereby, the electronic module 120 can be sealed.

In some examples, the isolator 130 may be elastic. In this case, after the first housing 110 and the second housing 200 are assembled, since the isolator 130 is elastic, it will rebound after being squeezed, so that the isolator 130 can be more closely attached to the second housing 200, thereby enhancing the sealing performance of the split-type medical apparatus 10.

In some examples, the isolator 130 may be adhesive. For example, the isolator 130 may be a gel-like adhesive component. In some examples, the isolator 130 may be bonded to the inner contour of the first housing 110. In this case, since the isolator 130 is bonded to the first housing 110, the junction portion between the isolator 130 and the first housing 110 can be closely attached, thereby preventing undesired substances from infiltrating into the electronic module 120 from the junction portion.

In some examples, the isolator 130 may cover the surface of the electronic module 120 and be bonded to the inner contour of the first housing 110. Thus, the sealing effect on the electronic module 120 can be enhanced, and in addition, the electronic module 120 can have good assembly stability.

In some examples, the isolator 130 may have a groove-shaped portion 131 (refer to Fig. 5A). In some examples, the groove-shaped portion 131 may match the mount 210. In some examples, the groove-shaped portion 131 matching the mount 210 may mean that the mount 210 can be accommodated in the groove-shaped portion 131. In other words, the isolator 130 may have a groove-shaped portion 131 for accommodating the mount 210.

In some examples, when the first housing 110 is assembled to the second housing 200, the mount 210 may be located in the groove-shaped portion 131 and the sensor 12 may be electrically connected to the electronic module 120 through the connection area 121. Thus, by positioning the mount 210 in the groove-shaped portion 131, the conductive assembly 2110 (to be described later) disposed on the mount 210 can be electrically connected to the connection area 121 located in the groove-shaped portion 131.

In some examples, the isolator 130 may cover the electronic module 120 in such a way that the connection area 121 is exposed from the groove-shaped portion 131. In this situation, when the first housing 110 is assembled to the second housing 200, the sensor 12 can be electrically connected to the connection area 121 through the conductive assembly 2120 accommodated in the base 212, thereby enabling the split-type medical apparatus 10 to obtain the analyte information.

In some examples, the isolator 130 may have a functional surface 132 facing away from the electronic module 120 (refer to Fig. 5A or Fig. 5B). In some examples, a protrusion 133 may be provided on the functional surface 132. In some examples, the protrusion 133 may surround the groove-shaped portion 131. In this case, through the interfitting of the protrusion 133 and the second housing 200, the groove-shaped portion 131 can be sealed, and thereby the electronic module 120 can be sealed.

In some examples, when the first housing 110 is assembled to the second housing 200, the isolator 130 may be located between the second housing 200 and the first housing 110.

In some examples, when the first housing 110 is assembled to the second housing 200, the protrusion 133 may deform in the direction from the second housing 200 toward the first housing 110. In this case, the protrusion 133 surrounding the groove-shaped portion 131 will be squeezed by the second housing 200 and thus deform, so that the isolator 130 can be more closely attached to the second housing 200 when the first housing 110 is assembled to the second housing 200, thereby achieving the sealing of the groove-shaped portion 131, that is, sealing the connection area 121, and further improving the sealing effect on the electronic module 120.

Hereinafter, for the convenience of describing the present disclosure, the above-mentioned protrusion 133 surrounding the groove-shaped portion 131 is referred to as a first protrusion 133.

In some examples, the protrusion 133 surrounding the groove-shaped portion 131 is referred to as a first protrusion 133.

In some examples, the groove-shaped portion 131 may be substantially groove-shaped.

In some examples, the groove-shaped portion 131 may include a bottom plate 134 (refer to Fig. 5A). In some examples, the bottom plate 134 may have a through-hole 1340 exposing the connection area 121 (refer to Fig. 5A). In other words, the through-hole 1340 may penetrate the bottom plate 134. Thereby, the connection area 121 can be exposed.

In some examples, the groove-shaped portion 131 may have a second protrusion 1341 surrounding the connection area 121 (refer to Fig. 5A). In some examples, when the first housing 110 is assembled to the second housing 200, the second protrusion 1341 may deform in the direction from the second housing 200 toward the first housing 110. In this case, when the first housing 110 is assembled to the second housing 200, the second protrusion 1341 will be squeezed by the mount 210 and thus deform, so that the second protrusion 1341 can be more closely attached to the mount 210, enabling the connection area 121 to be in a sealed space, that is, the interfitting between the second protrusion 1341 and the mount 210 can improve the sealing effect on the connection area 121, thereby improving the sealing effect on the electronic module 120.

In some examples, the second protrusion 1341 may be formed on the bottom plate 134 in a manner surrounding the connection area 121.

In some examples, when the first housing 110 is assembled to the second housing 200, the base 212 may press the second protrusion 1341, and the base 212 and the second protrusion 1341 may cooperate to form a sealed space. In this case, when the first housing 110 is assembled to the second housing 200, the second protrusion 1341 can be pressed by the base 212, so that the base 212 can be closely attached to the second protrusion 1341 to form a sealed space for accommodating the conductive assembly 2120 and the connection area 121.

As described above, in some examples, the mount 210 may include a positioning seat 211 and a base 212, and the positioning seat 211 may have a through-hole 2110 penetrating the second housing 200, and the sensor 12 may be positioned on the second housing 200 through the through-hole 2110. In this case, the sensor 12 can be disposed on the mount 210 through the through-hole 2110 of the positioning seat 211. When the first housing 110 is assembled to the second housing 200, the electrical connection between the sensor 12 and the electronic module 120 can be realized through the electrical connection between the conductive assembly 2120 and the connection area 121, and the through-hole 2120 penetrates the second housing 200 in the position of the positioning seat 211. In other words, the through-hole 2120 is independent of the sealed space, thereby isolating the undesired substances entering the split-type medical apparatus 10 through the through-hole 2120 from the sealed space, and further improving the protection effect on the electrical connection part of the split-type medical apparatus 10.

Hereinafter, for the convenience of describing the present disclosure, the above-mentioned groove-shaped portion 131 exposing the connection area 121 is referred to as a first groove-shaped portion 131. In other words, the groove-shaped portion 131 matching the mount 210 may be referred to as a first groove-shaped portion 131.

In some examples, the electronic assembly 100 may include a power module 140 (refer to Fig. 3). In other words, the split-type medical apparatus 10 may include a power module 140.

In some examples, the power module 140 may be disposed on the isolator 130. In some examples, the isolator 130 may have a second groove-shaped portion 135 for accommodating the power module 140 (refer to Fig. 5A).

In some examples, the power module 140 may be configured to supply energy to the electronic module 120.

In some examples, the protrusion 133 may surround the first groove-shaped portion 131 and the second groove-shaped portion 135. In other words, the first protrusion 133 may surround the first groove-shaped portion 131 and the second groove-shaped portion 135. In this case, the protrusion 133 surrounds the first groove-shaped portion 131 and the second groove-shaped portion 135. When the first housing 110 is assembled to the second housing 200, the protrusion 133 surrounding the first groove-shaped portion 131 and the second groove-shaped portion 135 will be squeezed by the second housing 200 and thus deform, so that the isolator 130 can be more closely attached to the second housing 200 when the first housing 110 is assembled to the second housing 200, and can seal the connection area 121 located in the first groove-shaped portion 131 and the power module 140 located in the second groove-shaped portion 135, thereby improving the sealing performance of the split-type medical apparatus 10.

In some examples, a third protrusion 136 may be provided on the functional surface 132 (refer to Fig. 5A). In some examples, the third protrusion 136 may be configured to be connected to the first protrusion 133 and isolate the first groove-shaped portion 131 and the second groove-shaped portion 135. In this case, when the first housing 110 is assembled to the second housing 200, through the interfitting of the first protrusion 133, the third protrusion 136 and the second housing 200, two independent sealed spaces respectively including the first groove-shaped portion 131 and the second groove-shaped portion 135 can be formed, and thereby can seal the connection area 121 and the mount 310 located in the first groove-shaped portion 131 and the power module 140 located in the second groove-shaped portion 135 respectively.

In some examples, the third protrusion 136 may be located within a closed shape formed by the first protrusion 133. In some examples, the third protrusion 136 may be connected to the first protrusion 133 to form two closed shapes. In some examples, the first groove-shaped portion 131 and the second groove-shaped portion 135 are respectively located in the two closed shapes. Thereby, when the first housing 110 is assembled to the second housing 200, the first groove-shaped portion 131 and the second groove-shaped portion 135 can be located in different sealed spaces.

In some examples, in the direction from the first housing 110 toward the second housing 200, the extension length of the third protrusion 136 may be the same as that of the first protrusion 133. In this case, when the first housing 110 is assembled to the second housing 200, the second housing 200 can simultaneously press the first protrusion 133 and the third protrusion 136, so that both the first protrusion 133 and the third protrusion 136 can be closely attached to the second housing 200.

In some examples, the second housing 200 may have at least one hole 220 for positioning the second housing 200. In some examples, when the first housing 110 is assembled to the second housing 200, the at least one hole 220 on the second housing 200 may be located on the side of the third protrusion 136 away from the second groove-shaped portion 135. In this case, since the hole(s) 220 is located on the side of the third protrusion 136 away from the second groove-shaped portion 135, when the first housing 110 is assembled to the second housing 200, the hole(s) 220 can be independent of the sealed space including the second groove-shaped portion 135, thereby isolating the undesired substances entering the split-type medical apparatus 10 through the hole(s) 220 from the sealed space including the second groove-shaped portion 135, and thus improving the sealing effect on the power module 140. It should be noted that the above-mentioned sentence that at least one hole 220 may be located on the side of the third protrusion 136 away from the second groove-shaped portion 135 means that all of the holes 220 for positioning the second housing 200 are located on the side of the third protrusion 136 away from the second groove-shaped portion 135.

In some examples, at least one fourth protrusion 137 matching the at least one hole 220 may be provided on the functional surface 132 (refer to Fig. 5A). In this case, the fourth protrusion(s) 137 can block the hole(s) 220, preventing undesired substances from entering the split-type medical apparatus 10 along the hole(s) 220, thereby further improving the sealing performance of the split-type medical apparatus 10.

In some examples, the shape of the fourth protrusion(s) 137 may match the shape of the hole(s) 220. For example, when the shape of the hole(s) 220 is circular, the shape of the fourth protrusion(s) 137 may also be circular.

In some examples, the radial dimension of the fourth protrusion(s) 137 may be not smaller than that of the hole(s) 220. In some examples, the circumferential dimension of the fourth protrusion(s) 137 may be not smaller than that of the hole(s) 220. Thus, the probability of undesired substances entering the split-type medical apparatus 10 can be reduced.

In some examples, the number of the fourth protrusion(s) 137 may be the same as that of the hole(s) 220. For example, as described above, when the first housing 110 has the hole 220a, the hole 220b, the hole 220c and the hole 220d, the isolator 130 may also be correspondingly provided with a fourth protrusion 137a, a fourth protrusion 137b, a fourth protrusion 137c and a fourth protrusion 137d corresponding to the four holes 220.

In some examples, when the first housing 110 is assembled to the second housing 200, the at least one fourth protrusion 137 may be squeezed and deformed in the direction from the second housing 200 toward the first housing 110. In this case, the fourth protrusion(s) 137 can block the hole(s) 220, preventing undesired substances from entering the split-type medical apparatus 10 along the hole(s) 220, thereby further improving the sealing performance of the split-type medical apparatus 10.

Fig. 6 is a flowchart showing a method for preparing an electronic assembly 100 according to an example of the present disclosure.

In some examples, the electronic assembly 100 may include an electronic module 120, an isolator 130 and a first housing 110.

As described above, the present disclosure also relates to a method for preparing the electronic assembly 100 (hereinafter may be referred to as the preparation method). In some examples, the electronic assembly 100 obtained by the preparation method of the present disclosure may be the electronic assembly 100 of the split-type medical apparatus 10 according to the first aspect of the present disclosure. In other words, the electronic assembly 100 obtained by the preparation method of the present disclosure may include the electronic module 120, the isolator 130 and the first housing 110 as described above.

In some examples, referring to Fig. 6, the preparation method may include: preparing the first housing 110 provided with the electronic module 120 (step S100), coupling the first housing 110 with a mold having a preset shape (step S200), supplying an injection molding material with a preset temperature to the mold (step S300), removing the mold after the injection molding material is cooled to obtain the isolator 130 (step S400), and obtaining the electronic assembly (step S500). In this case, in the electronic assembly 100 obtained by supplying the injection molding material to the mold, the isolator 130 can be closely bonded to the first housing 110 and seal the electronic module 120 disposed on the first housing 110. The preset shape of the mold matches the shape of the isolator 130, so that the injection molding material can form an isolator 130 with a desired structure after cooling, and the structure of the isolator 130 can improve the sealing performance of the electronic assembly 100 after assembly; at the same time, the isolator 130 can also play a role in stabilizing various electronic components of the electronic module 120.

As described above, in some examples, in step S100, the first housing 110 provided with the electronic module 120 may be prepared. In some examples, the electronic module 120 may be placed on the inner side of the first housing 110. In some examples, the electronic module 120 may be bonded to the inner side of the first housing 110.

In some examples, in step S200, the first housing 110 may be coupled with a mold having a preset shape. In some examples, the preset shape matches the shape of the isolator 130. In some examples, the inner contour of the mold may match the outer contour of the isolator 130. In this case, the injection molding material can form an isolator 130 with a desired structure after cooling, and the structure of the isolator 130 can improve the sealing performance of the electronic assembly 100 after assembly.

In some examples, the mold may have an injection port for supplying the injection molding material.

In some examples, in step S300, the injection molding material with a preset temperature may be supplied to the mold. In some examples, the preset temperature may be 150 degrees Celsius to 200 degrees Celsius. For example, the preset temperature may be 150 degrees Celsius, 160 degrees Celsius, 170 degrees Celsius, 180 degrees Celsius, 190 degrees Celsius, or 200 degrees Celsius, etc. In this case, during the injection molding process, the injection molding material with a low temperature can be injected into the mold, thereby further reducing the potential damage to the electronic assembly 100 during the injection molding process.

In some examples, the injection molding material may include silicone. In this case, the isolator 130 formed after the injection molding material is cooled can have high waterproofness and elasticity, thereby improving the sealing effect on the electronic module 120.

In some examples, the injection molding material may include silicone and a functional additive, and the functional additive may be an adhesion promoter that enhances bonding between silicone and the first housing 110. For example, the functional additive may be epoxy resin adhesive, neoprene adhesive, acrylic adhesive, polyurethane adhesive, etc.

In some examples, when the injection molding material includes silicone, the injection molding material may be supplied to the mold in a low-pressure manner. Low-pressure injection is an encapsulation process that injects the injection molding material into the mold with a low injection pressure (generally between 0.15 MPa and 4 MPa, where MPa is a unit of pressure). Thus, the potential damage to the electronic assembly 100 during the injection molding process can be reduced.

In some examples, in step S400, the mold may be removed after the injection molding material is cooled to obtain the isolator 130.

In some examples, the injection molding material may be cooled at normal room temperature. In some examples, the injection molding material may be cured to form a solid component after cooling. In some examples, the injection molding material may not adhere to the mold after cooling. Thus, the mold can be easily removed.

In some examples, the isolator 130 may cover the electronic module 120. Thus, the electronic module 120 can be sealed.

In some examples, the isolator 130 may cover the electronic module 120 in such a way that the connection area 121 of the electronic module 120 that needs to be electrically connected to the sensor 12 is exposed. Thus, the sensor 12 can be electrically connected to the connection area 121, thereby enabling the split-type medical apparatus 10 to obtain the analyte information, and the electronic module 120 can obtain the analyte information through the electrical connection.

In some examples, the hardness of the injection molding material after curing may be 10 Shore A to 60 Shore A. For example, the hardness may be 10 Shore A, 15 Shore A, 30 Shore A, 45 Shore A, or 60 Shore A. In this case, the isolator 130 can have high elasticity.

Generally speaking, the physical unit of the hardness of cured silicone is Shore hardness, which can be used to characterize the hardness of a material. The smaller the hardness, the softer the material. Silicone with a hardness of 10 Shore A has relatively soft elasticity, can be easily squeezed and rebound, providing an excellent sealing performance. Silicone with a hardness of 60 Shore A has elasticity, can be squeezed and rebound similarly, and it also offers greater durability and is less prone to wear when compared with silicone with lower hardness.

In some examples, in step S500, the electronic assembly 100 may be obtained. In some examples, the isolator 130 in the obtained electronic assembly 100 may seal the electronic module 120. In some examples, the electronic assembly 100 may be assembled with the second housing 200 and applied to the target.

In some examples, the electronic assembly 100 may further include a power module 140. In some examples, after the injection molding material is cured to obtain the isolator 130, the power module 140 may be installed in the second groove-shaped portion 135. In some examples, the power module 140 may include a battery compartment 141 and a battery 142 (refer to Fig. 3).

In the split-type medical apparatus 10 according to the present disclosure, the isolator 130 is attached to the first housing 110 and partially covers the electronic module 120, which can initially seal the electronic module 120, and since the isolator 130 has a groove-shaped portion 131 for exposing the connection area 121 and matching the mount 210 for disposing the sensor 12, when the first housing 110 is assembled to the second housing 200, the sensor 12 can be electrically connected to the connection area 121, thereby enabling the split-type medical apparatus 10 to obtain the analyte information. In addition, since the isolator 130 has a protrusion 133 surrounding the groove-shaped portion 131, when the first housing 110 is assembled to the second housing 200, the protrusion 133 surrounding the groove-shaped portion 131 will be squeezed by the second housing 200 and thus deform, so that the isolator 130 can be more closely attached to the second housing 200 when the first housing 110 is assembled to the second housing 200, thereby achieving the sealing effect on the groove-shaped portion 131, that is, sealing the connection area 121, and further improving the sealing effect on the electronic module 120.

In addition, through the method for preparing the electronic assembly 100, the first housing 110 and the isolator 130 can be closely bonded, and the structure of the isolator 130 can improve the sealing performance of the electronic assembly 100 after assembly; at the same time, the isolator 130 can also play a role in stabilizing various electronic components of the electronic module 120.

Although the present disclosure has been specifically described above with reference to the accompanying drawings and examples, it should be understood that the above description does not limit the present disclosure in any form. Those skilled in the art can make modifications and changes to the present disclosure as needed without departing from the essential spirit and scope of the present disclosure, and these modifications and changes all fall within the scope of the present disclosure.

## Claims

1. A split-type medical apparatus, **characterized in that** the split-type medical apparatus comprises a first housing, an electronic module and an isolator disposed on the first housing, a second housing detachably assembled with the first housing, and a sensor disposed on the second housing,
the second housing includes a mount for disposing the sensor which could be placed subcutaneously;
the isolator has a groove-shaped portion matching the mount and a functional surface facing away from the electronic module, with a protrusion surrounding the groove-shaped portion provided on the functional surface;
the electronic module includes a connection area for electrical connection with the sensor, and the isolator is configured to be attached to the first housing and cover the electronic module in such a way that the connection area is exposed from the groove-shaped portion;
when the first housing is assembled to the second housing, the isolator is located between the second housing and the first housing, the protrusion deforms in the direction from the second housing toward the first housing, and the mount is located in the groove-shaped portion and the sensor is electrically connected to the electronic module through the connection area.

2. The split-type medical apparatus according to claim 1, **characterized in that** the protrusion surrounding the groove-shaped portion is referred to as a first protrusion, the groove-shaped portion has a second protrusion surrounding the connection area, and when the first housing is assembled to the second housing, the second protrusion deforms in the direction from the second housing toward the first housing.

3. The split-type medical apparatus according to claim 1 or 2, **characterized in that** the split-type medical apparatus comprises a power module configured to supply energy to the electronic module, the groove-shaped portion matching the mount is referred to as a first groove-shaped portion, the isolator has a second groove-shaped portion configured to accommodate the power module, and the protrusion surrounds the first groove-shaped portion and the second groove-shaped portion.

4. The split-type medical apparatus according to claim 3, **characterized in that** the protrusion surrounding the groove-shaped portion is referred to as a first protrusion, a third protrusion connected to the first protrusion and configured to isolate the first groove-shaped portion and the second groove-shaped portion is provided on the functional surface, and in the direction from the first housing toward the second housing, the extension length of the third protrusion is the same as that of the first protrusion.

5. The split-type medical apparatus according to claim 2, **characterized in that** the mount includes a positioning seat and a base, the positioning seat has a through-hole penetrating the second housing, and the sensor is positioned on the second housing through the through-hole;
the base is configured to accommodate a conductive assembly electrically connected to the sensor, the sensor is electrically connected to the connection area through the conductive assembly, and when the first housing is assembled to the second housing, the base presses the second protrusion, and the base and the second protrusion cooperate to form a sealed space.

6. The split-type medical apparatus according to claim 4, **characterized in that** the second housing has at least one hole for positioning the second housing, and when the first housing is assembled to the second housing, the at least one hole is located on the side of the third protrusion away from the second groove-shaped portion.

7. The split-type medical apparatus according to claim 6, **characterized in that** at least one fourth protrusion matching the at least one hole is provided on the functional surface, and when the first housing is assembled to the second housing, the at least one fourth protrusion deforms in the direction from the second housing toward the first housing.

8. The split-type medical apparatus according to claim 1, **characterized in that** the isolator is elastic and is bonded to the inner contour of the first housing.

9. A method for preparing an electronic assembly, **characterized in that** the electronic assembly includes the electronic module, the isolator and the first housing according to any one of claims 1 to 8, and the method for preparing the electronic assembly comprises:
preparing the first housing provided with the electronic module;
coupling the first housing with a mold having a preset shape, the preset shape matching the shape of the isolator;
supplying an injection molding material with a preset temperature to the mold;
removing the mold after the injection molding material is cooled to obtain the isolator; and
obtaining the electronic assembly.

10. The method for preparing the electronic assembly according to claim 9, **characterized in that** the injection molding material includes silicone.

11. The method for preparing the electronic assembly according to claim 9, **characterized in that** the preset temperature is 150 degrees Celsius to 200 degrees Celsius.

12. The method for preparing the electronic assembly according to claim 9, **characterized in that** the hardness of the injection molding material after curing is 10 Shore A to 60 Shore A.
